# EUROPEAN PATENT SPECIFICATION

(11) **EP 3 330 244 B1**
(45) Date of publication and mention of the grant of the patent: **18.08.2021**
(21) Application number: 15899132.3
(22) Date of filing: 21.09.2015
(51) Int. Cl.: C07C 17/23, C07C 17/383, C07C 21/18

(54) **PRODUCTION METHOD FOR 2,3,3,3-TETRAFLUOROPROPENE**
HERSTELLUNGSVERFAHREN FÜR 2,3,3,3-TETRAFLUORPROPEN
PROCÉDÉ DE PRODUCTION DU 2,3,3,3-TÉTRAFLUOROPROPÈNE

(30) Priority: 30.07.2015 CN 201510459642
(43) Date of publication of application: 06.06.2018
(73) Proprietor: Zhejiang Quzhou Juxin Fluorine Chemical Co., Ltd., Quzhou, Zhejiang 324004 (CN); Zhejiang Quhua Fluor-Chemistry Co., Ltd., Quzhou, Zhejiang 324004 (CN)
(72) Inventor: LEI, Jun, Ningbo Zhejiang 315000 (CN); WANG, Aiguo, Quzhou Zhejiang 324000 (CN); YANG, Bo, Quzhou Zhejiang 324000 (CN); ZHANG, Yan, Quzhou Zhejiang 324000 (CN); ZHOU, Huadong, Quzhou Zhejiang 324004 (CN); ZHAO, Yang, Quzhou Zhejiang 324000 (CN); ZHU, Yi, Quzhou Zhejiang 324000 (CN); XIA, Haili, Quzhou Zhejiang 324000 (CN); SHI, Haojin, Quzhou Zhejiang 324004 (CN)
(74) Representative: Lang, Christian
(86) International application number: PCT/CN2015/000655
(87) International publication number: WO 2017/015779

(56) References cited:
- WO-A1-2009/084703
- WO-A1-2009/118632
- WO-A1-2009/138764
- WO-A2-2008/030440
- CN-A- 102 026 947
- CN-A- 102 267 869
- FR-A1- 2 935 703
- KNUNYANTS I L ET AL: "Reactions of fluoro olefins. XIII. Catalytic hydrogenation of perfluoro olefins", IZVESTIYA AKADEMII NAUK SSSR, SERIYA KHIMICHESKAYA, INSTITUT ORGANICHESKOI KHIMII IM. N. D. ZELINSKOGO ROSSIISKOI AKADEMII, RU, January 1960 (1960-01), pages 1312-1318, XP002548816, ISSN: 0002-3353

## Description

The invention relates to a method for preparing a fluorine-containing olefin, more particularly to a method for preparing 2,3,3,3-tetrafluoropropene.

As a global hot topic, fluorinated refrigerant substitutes are required to have an ozone depression potential (ODP) of 0, a global warming potential (GWP) as low as possible, and an atmospheric lifetime as short as possible. As a first generation of refrigerant, chlorofluorocarbons such as dichlorodifluoromethane (CCl₂F₂, CFC-12) have been discarded because the ODP thereof are approximately 1. As a first and second generations of transitional refrigerant substitutes, hydrogen chlorofluorocarbons 1-chloro-2-fluoro-methane (CHClF₂, HCFC-22) are also being discarded because of the ODP thereof are larger than 0. As a third generation of refrigerant, chlorofluorocarbon 1,1,1,2-tetrafluoroethane (CF₃CH₂F, HFC-134a) is widely applied in mobile air conditioners, household appliances, and industrial and commercial refrigeration devices, and can be also applied in foam, extinguishing, aerosol, and cleaning industries. However, HFC-134a has relatively high greenhouse effect (GWP=1300), long atmospheric lifetime, and undue use thereof may result in global warming, thus HFC-134a is to be discarded.

As refrigerant containing a single working fluid, 2,3,3,3-tetrafluoropropene (HFO-1234yf) features superb environmental parameters, GWP=4, ODP=0, life cycle climate performance (LCCP) being lower than HFC-134a, and atmospheric decomposed products being the same as those of HFC-134a, and more excellent systematic performance than HFC-134a. When HFO-1234yf is selected to substitute HFC-134A, car manufactures are able to use the original mobile air conditioning (MAC) system. Thus, HFO-1234yf is supposed to be a new generation of mobile refrigerant substitutes with potentials and has been accepted by car manufacturers in east Europe and has been gradually applied since 2011.

Because of the excellent performance and wide range of uses the synthesis of HFO-1234yf attracts more and more attentions. Early in 1950s, the preparation of HFO-1234fy was disclosed by DowDuPont chemical company. HFO-1234yf is primarily used to prepare polymerized monomers and comonomer. In the early 1980s, Daikin company started technology development in HFO-1234yf. In 21st century, Honeywell and DuPont company dedicated their work in developing HFO-1234yf and applied a large amount of patents.

Methods for preparing HFO-1234yf are primarily based on three routings: trifluoropropene process, hexafluoropropene process, and tetrachloropropene process, while other preparation methods are all branches of these three routings. The trifluoropropene process adopts a four-step synthetic route for HFO-1234y and imposes high requirements on the chlorination reactor. Much residual solution is produced in the two saponification reactions, the chlorination reactor is seriously corroded, the service life of the catalyst is short, the total yield is low, and the synthesis cost is high. The tetrachloropropene process is able to synthesize HFO-1234yf by two steps. The second step reaction features high temperature, low conversion rate of the catalyst, and relatively high energy consumption.

The method that utilizes hexafluoropropylene (HFP) and hydrogen (H₂) as raw materials to synthesize HFO-1234yf generally requires four-step reactions, i. e., two-step hydrogenation reaction and two-step dehydrofluorination reaction. Thus, the hexafluoropropene process features multiple process steps, low efficiency, large investment in apparatuses, and high production cost.

Chinese Patent publication number CN102026947A, disclosed on April 20, 2011, titled as method for preparing 2,3,3,3-tetrafluoropropene. The method includes: a) allowing 1,1,1,2,3,3-hexafluoropropylene (HFP) to contact with (H₂) in the presence of a hydrogenation catalyst to produce 1,1,1,2,3,3-hexafluoropropane (HFC-236ea) to yield 1,1,1,2,3,3-hexafluoropropane (HFC-236ea); b) dehydrochlorinating HFC-236ea to yied 1,1,1,2,3-pentafluoropropene (HFO-1225ye); c) allowing HFO-1225ye to contact with H₂ in the presence of a hydrogenation catalyst to produce 1,1,1,2,3-pentafluoropropane (HFC-245eb); and d) dehydrochlorinating HFC-245eb to yield HFO-1234yf. The method is carried out by four steps, the reaction routing is long, a total yield is not high, and the investment in the apparatus is great.

Chinese patent publication number CN101544536A, disclosed on September 30, 2009, titled as a method for preparing fluorinated olefin. The invention relates to a method for preparing a fluorinated olefin, and specifically provides a method for preparing 1,1,1,2-tetrafluoropropene and/or 1,1,1,2,3-pentafluoropropene using four units of single series, and the unit operation includes: 1) hydrogenating raw materials including hexafluoropropylene and any selected cycled 1,1,1,2,3-pentafluoropropene; 2) separating anticipated intermediate fluorinated alkanes, and the fluorinated alkanes are 1,1,1,2,3,3-hexafluoropropane and/or 1,1,1,2,3-pentafluoropropane; 3) dehydrofluorinating of the intermediated fluorinated alkanes for yielding anticipated 1,1,1,2,3-pentafluoropropene and/or 1,1,1,2-tetrafluoropropene, and separating anticipated products, and optionally cycling 1,1,1,2,3-pentafluoropropene. The two-step hydrogenation of the invention is performed in a single reactor and the two-step dehydrofluorination is performed in a signal reactor, thus, the catalysts are highly required, and much by-product is produced.

Chinese patent publication number CN101671229A, disclosed on March 17, 2010, titled as a method for preparing a fluorinated compound. The method includes the following steps: 1) hydrogenating hexafluoropropylene to yield 1,1,1,2,3,3-hexafluoropropane; 2) dehydrofluorinating 1,1,1,2,3,3-hexafluoropropane to yield 1,2,3,3,3-hexafluoro-1-propene; 3) hydrogenating 1,2,3,3,3-hexafluoro-1-propene to yield 1,1,1,2,3-pentafluoropropane; 4) dehydrofluorinating 1,1,1,2,3-pentafluoropropane to yield 1,2,3,3,3-hexafluoro-1-propene. A mixture of water and potassium hydroxide was utilized in step 2) and 4) at a temperature of between 110 and 180°C. Potassium hydroxide accounts for between 58 and 86 wt. % of the mixture. The invention is inferior in long process routing, liquid phase where dehydrofluorination is performed, much three wastes is produced, low efficiency, and serious corrosion inside the reactor.

WO 2009/084703 A1 recites a method for preparing 2,3,3,3-tetrafluoropropene from hexafluoropropene through hydrogenation and dehydrofluorination. WO 2009/118632 A1 recites a method for preparing 2,3,3,3-tetrafluoropropene from hexafluoropropylene. "Reactions of fluoro olefins. XIII. Catalytic hydrogenation of perfluoro olefins", IZVESTIYAAKADEMII NAUK SSSR, SERIYA KHIMICHESKAYA, INSTITUT ORGANICHESKOI KHIMII IM. N. D. ZELINSKOGO ROSSIISKOI AKADEMII, RU, January 1960 (1960-01), pages 1312-1318, recites a series of chemical reactions of hexafluoropropene. WO 2008/030440 A2 recites a method of producing 2,3,3,3- tetrafluoropropene from 1,2,3,3,3-pentafluoropropene.

In view of the above-described problems, it is one objective of the invention to provide a method for preparing 2,3,3,3-tetrafluoropropene that is shorter, more efficient, and less expensive, and produces the desired material with more selectivity and in greater yield.

To achieve the above objective, in accordance with one embodiment of the invention, there is provided a method for preparing 2,3,3,3-tetrafluoropropene. The method comprises:
a) introducing hexafluoropropylene and hydrogen to a first reactor to allow hexafluoropropylene to react with hydrogen in the presence of a first catalyst and a second catalyst to obtain a first mixture comprising 1,2,3,3,3-pentafluoropropene, 1,1,1,2,3,3-hexafluoropropane, hydrogen fluoride, and hexafluoropropylene, in which a molar ratio of hexafluoropropylene to hydrogen is between 1: 0.95 and 1: 0.99, a space velocity is between 200 and 1000 h⁻¹, and a reaction temperature is between 50 and 400°C;
b) washing and drying the first mixture obtained from a), and introducing the treated first mixture to a first distillation column to obtain 1,1,1,2,3,3-hexafluoropropane in a bottom of the first distillation column and 1,2,3,3,3-pentafluoropropene and hexafluoropropylene at a top thereof; recycling 1,1,1,2,3,3-hexafluoropropane to the first reactor, and introducing 1,2,3,3,3-pentafluoropropene and hexafluoropropylene to a second distillation column to yield hexafluoropropylene at a top of the second distillation column and 1,2,3,3,3-pentafluoropropene at a bottom thereof; recycling hexafluoropropylene to the first reactor;
c) introducing 1,2,3,3,3-pentafluoropropene obtained from b) and hydrogen to a second reactor and allowing 1,2,3,3,3-pentafluoropropene to react with hydrogen in the presence of a third catalyst and a fourth catalyst, to obtain a second mixture comprising 1,1,1,2,3-pentafluoropropane, 2,3,3,3-tetrafluoropropene, HF, and H₂, in which a molar ratio of hydrogen to 1,2,3,3,3-pentafluoropropene is between 1: 0.95 and 1: 0.99, a space velocity is between 300 and 2000 h⁻¹, and a reaction temperature is between 80 and 500°C; and
d) washing and drying the second mixture obtained from c), and introducing the second mixture to a third distillation column to yield 1,1,1,2,3-pentafluoropropane at a bottom thereof; and recycling 1,1,1,2,3-pentafluoropropane to the second reactor, to yield 2,3,3,3-tetrafluoropropene at a top of the third distillation column.

In a class of this embodiment, the space velocity in a) is between 400 and 800 h⁻¹, and the reaction temperature is between 100 and 300°C.

In a class of this embodiment, the space velocity in c) is between 600 and 1500 h⁻¹, and the reaction temperature is between 120 and 400°C.

In a class of this embodiment, the first catalyst and the second catalyst in the first reactor are respectively filled in an upper section and a lower section of the first reactor. The first catalyst in the upper section of the first reactor is Pd/C, and Pd accounts for between 0.1 and 1 wt. %. The second catalyst in the lower section of the first reactor is chromium oxide.

In a class of this embodiment, the third catalyst and the fourth catalyst in the second reactor are respectively filled in an upper section and a lower section of the second reactor. The third catalyst in the upper section of the second reactor is Pd/Al₂O₃, and Pd accounts for between 0.2 and 1.5 wt. %. The fourth catalyst in the lower section of the second reactor comprise between 80 and 90 wt. % of chromium oxide and between 10 and 20 wt. % of zinc oxide.

In a class of this embodiment, both the first reactor and the second reactor are adiabatic reactors.

Both the first reactor and the second reactor can be divided into two sections with each section filled with different catalysts. The raw materials hexafluoropropylene and H₂ after being heated by a preheater enter the first reactor for reaction under the action of the catalysts in the upper section and the lower section. The dose of hexafluoropropylene is slightly excessive while H₂ is completely converted, thus, an obtained mixture includes 1,1,1,2,3,3-hexafluoropropane produced from the reaction, and a small amount of non-reacted hexafluoropropylene. The mixture then enters the lower section of the reactor where dehydrofluorination of 1,1,1,2,3,3-hexafluoropropane in a gas phase is performed to yield a mixture including 1,1,1,2,3,3-hexafluoropropane, 1,1,1,2,3-pentafluoropropene, HF, and a small amount of hexafluoropropylene. The mixture is then separated, the non-reacted 1,1,1,2,3,3-hexafluoropropane and hexafluoropropylene are returned to the first reactor, and 1,1,1,2,3-pentafluoropropene and the fresh H₂ are preheated by a preheater and introduced to the second reactor for reaction under the action of the catalysts of the upper and the lower sections, during which, the dose of H₂ is slightly excessive and 1,1,1,2,3-pentafluoropropene is completely converted, a resulting mixture includes 2,3,3,3-tetrafluoropropene, 1,1,1,2,3-pentafluoropropane, HF, and a small amount of H₂. The mixture is then separated to yield the product 2,3,3,3-tetrafluoropropene, while the non-reacted 1,1,1,2,3-pentafluoropropane is returned to the second reactor and the small amount of H₂ is discharged.

Hydrogenation of hexafluoropropylene and dehydrofluorination of 1,1,1,2,3,3-hexafluoropropane are performed in the first reactor. The hydrogenation of hexafluoropropylene is a strong exothermic reaction. The activity of the catalyst and the selectivity of the products are greatly influenced by the reaction temperature, and too high the temperature may result in coking and deactivation of the catalyst for hydrogenation. The temperature of the hydrogenation is lower than the temperature of dehydrofluorination, the heat quantity of the lower section is partially supply of heat quantity produced by the hydrogenation of the upper section, and the heat quantity of the hydrogenation of the upper section is carried away by the excessive 1,1,1,2,3,3-hexafluoropropane. The temperature of the upper section of the first reactor is controlled between 50 and 150°C, and preferably between 80 and 120°C, and the temperature of the lower section is between 250 and 400°C. The higher the space velocity is, the more the materials that the unit catalyst surface contacts are, and the higher the loading of the reaction is. Thus, based on comprehensive consideration, the selected space velocity is between 200 and 1000 h⁻¹, preferably between 400 and 800 h⁻¹. To make H₂ totally converted for avoiding subsequent separation problem of H₂, the dose of hexafluoropropylene is slightly excessive and the excessive hexafluoropropylene can be returned to the first reactor; and the molar ratio of hexafluoropropylene to hydrogen is between 1: 0.95 and 1:0.99.

Hydrogenation of 1,1,1,2,3-pentafluoropropene and dehydrofluorination of 1,1,1,2,3-pentafluoropropane are performed in the second reactor. Similar to the reactions in the first reactor, a part of the heat quantity of the lower section is also supplied by the heat quantity produced in the hydrogenation in the upper section. 1,1,1,2,3-pentafluoropropene is made completely converted, thus avoiding the subsequent problems of difficult separation of 1,1,1,2,3-pentafluoropropene and 2,3,3,3-tetrafluoropropene. The temperature of the upper section of the second first reactors controlled between 80 and 200°C, preferably between 100 and 150°C, and the temperature of the lower section is controlled at between 300 and 500°C, and the space velocity is controlled between 300 and 2000 h⁻¹, preferably between 600 and 1500 h⁻¹, and a molar ratio of H₂ to 1,1,1,2,3-pentafluoropropene is between 1: 0.95 and 1: 0.99.

The upper sections of the first reactor and the second reactor are filled with the precious metal Pd catalyst. As the loading amount of the precious metal is too low, the catalytic activity is not enough, and a best balance point exists among the amount of Pd and the catalytic activity. The selection of the carrier is critical for the catalyst. The hydrogenation of HFP is easily carried out, the activated carbon is selected as the catalyst because of its high specific area, which is beneficial to load catalytic substance with small amount but high dispersion degree and to prepare catalyst of high catalytic activity. The hydrogenation of 1,1,1,2,3-pentafluoropropene is difficult, the Al₂O₃ carrier has relatively strong acidity, the active component Pd is well dispersed on the carrier. Al₂O₃ carrier has high mechanical strength, and the catalyst has long service life. It is found from the experiment that the upper section of the first reactor is filled with the Pd/C catalyst and Pd accounts for between 0.1 and 1 wt. %, and the lower section of the second first reactors filled with the Pd/Al₂O₃ catalyst and Pd accounts for between 0.2 and 1.5 wt. %. Pretreatment of the catalyst can be conducted in other reactors.

The catalysts in the lower sections of the first reactor and the second reactor are those including chromium oxide as the active component. The catalyst of the lower section of the first reactor is the pure chromium oxide, and the catalyst of the lower section of the second reactor comprises between 80 and 90 wt. % of chromium oxide and between 10 and 20 wt. % of zinc oxide and is prepared as follows: mixing nitrates of chromium and zinc according to a certain ratio to prepare a diluted solution of a certain concentration, adding a precipitant for reaction, performing filtration, washing by water, desiccation, calcination, granulation, and tablet pressing to prepare a precursor, fluorinating the precursor to yield the catalyst. The pretreatment of the catalyst can be conducted in other reactors.

Both the first reactor and the second reactor adopt the adiabatic type or the isothermal type, preferably adopting the adiabatic type. And the material of the reactors can adopt the carbon steel or the stainless steel.

Advantages of a method for preparing 2,3,3,3-tetrafluoropropene according to embodiments of the invention are summarized as follows:
1. The two-step gas phase route is adopted, the procedure is simple, and there are few byproducts produced.
2. The conversion rate and the selectivity are high, the conversion rate of hexafluoropropylene is higher than 99%, and the conversion rate of 1,1,1,2,3-pentafluoropropene is 100%. Both the selectivity of 1,1,1,2,3,3-hexafluoropropane and 1,1,1,2,3-pentafluoropropane are 100%.
3. H₂ of the first reactor is totally converted and 1,1,1,2,3-pentafluoropropene of the second first reactors totally converted, so that the separation problems of the non-reacted H₂ and 1,1,1,2,3-pentafluoropropene are solved.
4. The heat quantity produced in the hydrogenation is fully utilized by the dehydrofluorination, thus, the heat quantity is comprehensively utilized, and the energy consumption is reduced.

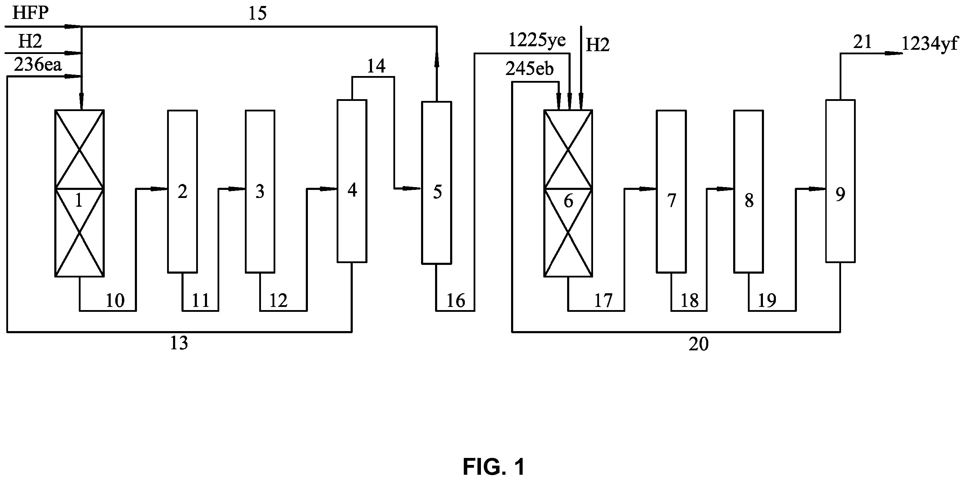

The invention is described hereinbelow with reference to accompanying drawings, in which FIG. 1 is a flow chart illustrating a method for preparing 2,3,3,3-tetrafluoropropene according to one embodiment of the invention.

In the drawings, the following reference numbers are used: 1. First reactor; 2. First alkaline washing column; 3. First drying column; 4. First distillation column; 5. Second distillation column; 6. Second reactor; 7. Second alkaline washing column; 8. Second drying column; 9. Third distillation column; and 10-21. Pipelines.

As shown in FIG. 1, raw materials hexafluoropropylene and H₂ are introduced to a first reactor for reaction to yield a mixture comprising 1,1,1,2,3,3-hexafluoropropane, 1,1,1,2,3-pentafluoropropene, HF, and HFP at an outlet of the first reactor. The mixture is introduced via a pipeline 10 to a first alkaline washing column 2 for removing HF therefrom. A resulting mixture is introduced to a first drying tower 3 via a pipeline 11 for drying, then to a first distillation column 4 via a pipeline 12. 1,1,1,2,3,3-hexafluoropropane is obtained at a bottom of the first distillation column 4 and cycled to the first reactor via a pipeline 13, and 1,1,1,2,3-pentafluoropropene and HFP are obtained at a top of the first distillation column 4 and introduced to a second distillation column 5 via a pipeline 14. HEP was obtained at a top of the second distillation column 5 and cycled to the first reactor via a pipeline 15; and 1,1,1,2,3-pentafluoropropene was obtained at a bottom of the second distillation column 5 and introduced to a second reactor via a pipe 16. In the meanwhile, fresh H₂ is added to the second reactor for reaction to yield a mixture comprising 1,1,1,2,3-pentafluoropropane, 2,3,3,3-tetrafluoropropene, HF, and H₂ at an outlet of the second reactor. The mixture is than introduced to a second alkaline washing column 7 via a pipe 17 for removing HF therefrom. A resulting mixture is introduced to a second drying column 8 via a pipe 18, and then to a third distillation column 9 via a pipe 19. 1,1,1,2,3-pentafluoropropane is obtained at a bottom of the third distillation column 9 and cycled to the second reactor via a pipe 20, product 2,3,3,3-tetrafluoropropene is obtained at a top of the third distillation column 9, and H₂ is discharged as a non-condensed gas.

For further illustrating the invention, experiments are described below. It should be noted that the following examples are intended to describe and not to limit the invention.

### Example 1

200 mL of a Cr₂O₃ catalyst was added with HF for fluorination at a temperature of 350°C for 30 hrs to yield an activated Cr₂O₃ catalyst, which was then added to a lower section of the first reactor (adiabatic reactor made of carbon steel). 150 mL of a Pd/C catalyst (Pd accounts for 0.1 wt. %) was pretreated with a mixed gas comprising H₂ and N₂ (a molar ratio of H₂ to N₂ is 1:19) at a space velocity of 1200 mL g⁻¹ (catal.) h⁻¹ at a temperature of 350°C for 15 hrs, then the Pd/C catalyst after treatment was filled in an upper section of the first reactor. The upper section of the first reactor was heated to a temperature of 50°C, and the lower section thereof was heated to the temperature of 300°C. Thereafter, hexafluoropropylene and H₂ with a molar ratio of 1:0.95 was introduced to the first reactor at a space velocity of 300 h⁻¹ for reaction, and products obtained from an outlet of the first reactor were washed by an alkaline and then samples were collected for analysis, results of which are listed in Table 1-1.

**Table 1-1 Data analysis of organic substances at an outlet of a first reactor**

| Components | 1,1,1,2,3-pent afluoropropen e | 1,1,1,2,3,3-he xafluoropropa ne | HFP |
|---|---|---|---|
| Contents (wt. %) | 36.2 | 63.5 | 0.3 |

200 mL of a catalyst comprising Cr₂O₃ and ZnO₂ (90 wt. % of Cr₂O₃ and 10 wt. % of ZnO₂) was added with HF for fluorination at a temperature of 350°C for 30 hrs to yield an activated catalyst comprising Cr₂O₃ and ZnO₂, which was then added to a lower section of the second reactor (adiabatic reactor made of carbon steel). 180 mL of a Pd/Al₂O₃ catalyst (Pd accounts for 0.3 wt. %) was pretreated with a mixed gas comprising H₂ and N₂ (a molar ratio of H₂ to N₂ is 1:19) at a space velocity of 1200 mL g⁻¹ (catal.) h⁻¹ at a temperature of 350°C for 15 hrs, then the Pd/Al₂O₃ catalyst after treatment was filled in an upper section of the second reactor. The upper section of the second reactor was heated to a temperature of 100°C, and the lower section thereof was heated to the temperature of 320°C. Thereafter, 1,1,1,2,3-pentafluoropropene and H₂ obtained from the first reactor were introduced to the second reactor for reaction with a molar ratio of H₂ to 1,1,1,2,3-pentafluoropropene of 1:0.96 and a space velocity of 300 h⁻¹, and products obtained from an outlet of the second reactor were washed by an alkaline and then samples were collected for analysis, results of which are listed in Table 1-2.

**Table 1-2 Data analysis of organic substances at an outlet of a second reactor**

| Components | 2,3,3,3-tetrafl uoropropene | 1,1,1,2,3-pent afluoropropan e |
|---|---|---|
| Contents (wt. %) | 28.3 | 71.7 |

### Example 2

The activation of the Cr₂O₃ catalyst and the pretreatment of the Pd/C catalyst were the same as Example 1. 200 mL of the activated Cr₂O₃ catalyst was filled in a lower section of a first reactor (adiabatic reactor made of carbon steel), and 150 mL of the pretreated Pd/C catalyst (Pd accounts for 0.3 wt. %) was filled in an upper section of the first reactor. The upper section of the first reactor was heated to the temperature of 80°C, and the lower section of the first reactor was heated to the temperature of 280°C. Hexafluoropropylene and H₂ were introduced to the first reactor at a molar ratio of hexafluoropropylene to H₂ of 1:0.95 at a space velocity of 200 h⁻¹ for reaction, and products obtained from an outlet of the first reactor were washed by an alkaline and then samples were collected for analysis, results of which are listed in Table 2-1.

**Table 2-1 Data analysis of organic substances at an outlet of a first reactor**

| Components | 1,1,1,2,3-pent afluoropropen e | 1,1,1,2,3,3-he xafluoropropa ne | HFP |
|---|---|---|---|
| Contents (wt. %) | 42.5 | 57.3 | 0.2 |

The activation of the catalyst comprising Cr₂O₃ and ZnO₂ and the pretreatment of the Pd/Al₂O₃ catalyst were the same as Example 1. 200 mL of the activated catalyst comprising Cr₂O₃ and ZnO₂ (88 wt. % of Cr₂O₃ and 12 wt. % of ZnO₂) was filled in a lower section of the second reactor (adiabatic reactor made of carbon steel), and 180 mL of the pretreated Pd/Al₂O₃ catalyst (containing 0.5 wt. % of Pd) was filled in an upper section of the second reactor. The upper section of the second reactor was heated to the temperature of 120°C, and the lower section thereof was heated to the temperature of 300°C. Thereafter, 1,1,1,2,3-pentafluoropropene and H₂ obtained from the first reactor were introduced to the second reactor for reaction with a molar ratio of H₂ to 1,1,1,2,3-pentafluoropropene of 1:0.95 and a space velocity of 800 h⁻¹, and products obtained from an outlet of the second reactor were washed by an alkaline and then samples were collected for analysis, results of which are listed in Table 2-2.

**Table 2-2 Data analysis of organic substances at an outlet of a second reactor**

| Components | 2,3,3,3-tetrafluorop ropene | 1,1,1,2,3-pentafluor opropane |
|---|---|---|
| Contents (wt. %) | 31.7 | 68.3 |

### Example 3

The activation of the Cr₂O₃ catalyst and the pretreatment of the Pd/C catalyst were the same as Example 1. 200 mL of the activated Cr₂O₃ catalyst was filled in a lower section of a first reactor (adiabatic reactor made of carbon steel), and 150 mL of the pretreated Pd/C catalyst (containing 0.5 wt. % of Pd) was filled in an upper section of the first reactor. The upper section of the first reactor was heated to the temperature of 100°C, and the lower section of the first reactor was heated to the temperature of 320°C. Hexafluoropropylene and H₂ were introduced to the first reactor at a molar ratio of hexafluoropropylene to H₂ of 1:0.97 at a space velocity of 800 h⁻¹ for reaction, and products obtained from an outlet of the first reactor were washed by an alkaline and then samples were collected for analysis, results of which are listed in Table 3-1.

**Table 3-1 Data analysis of organic substances at an outlet of a first reactor**

| Components | 1,1,1,2,3-pent afluoropropen e | 1,1,1,2,3,3-he xafluoropropa ne | HFP |
|---|---|---|---|
| Contents (wt. %) | 54.3 | 45.6 | 0.1 |

The activation of the catalyst comprising Cr₂O₃ and ZnO₂ and the pretreatment of the Pd/Al₂O₃ catalyst were the same as Example 1. 200 mL of the activated catalyst comprising Cr₂O₃ and ZnO₂ (88 wt. % of Cr₂O₃ and 12 wt. % of ZnO₂) was filled in a lower section of the second reactor (adiabatic reactor made of carbon steel), and 180 mL of the pretreated Pd/Al₂O₃ catalyst (containing 0.8 wt. % of Pd) was filled in an upper section of the second reactor. The upper section of the second reactor was heated to the temperature of 150°C, and the lower section thereof was heated to the temperature of 400°C. Thereafter, 1,1,1,2,3-pentafluoropropene and H₂ obtained from the first reactor were introduced to the second reactor for reaction with a molar ratio of H₂ to 1,1,1,2,3-pentafluoropropene of 1:0.97 and a space velocity of 800 h⁻¹, and products obtained from an outlet of the second reactor were washed by an alkaline and then samples were collected for analysis, results of which are listed in Table 3-2.

**Table 3-2 Data analysis of organic substances at an outlet of a second reactor**

| Components | 2,3,3,3-tetrafluorop ropene | 1,1,1,2,3-pentafluor opropane |
|---|---|---|
| Contents (wt. %) | 56.8 | 43.2 |

### Example 4

The activation of the Cr₂O₃ catalyst and the pretreatment of the Pd/C catalyst were the same as Example 1. 200 mL of the activated Cr₂O₃ catalyst was filled in a lower section of a first reactor (adiabatic reactor made of carbon steel), and 150 mL of the pretreated Pd/C catalyst (containing 0.8 wt. % of Pd) was filled in an upper section of the first reactor. The upper section of the first reactor was heated to the temperature of 120°C, and the lower section of the first reactor was heated to the temperature of 310°C. Hexafluoropropylene and H₂ were introduced to the first reactor at a molar ratio of hexafluoropropylene to H₂ of 1:0.97 at a space velocity of 600 h⁻¹ for reaction, and products obtained from an outlet of the first reactor were washed by an alkaline and then samples were collected for analysis, results of which are listed in Table 4-1.

**Table 4-1 Data analysis of organic substances at an outlet of a first reactor**

| Components | 1,1,1,2,3-pent afluoropropen e | 1,1,1,2,3,3-he xafluoropropa ne | HFP |
|---|---|---|---|
| Contents (wt. %) | 57 | 42.8 | 0.2 |

The activation of the catalyst comprising Cr₂O₃ and ZnO₂ and the pretreatment of the Pd/Al₂O₃ catalyst were the same as Example 1. 200 mL of the activated catalyst comprising Cr₂O₃ and ZnO₂ (88 wt. % of Cr₂O₃ and 12 wt. % of ZnO₂) was filled in a lower section of the second reactor (adiabatic reactor made of stainless steel), and 180 mL of the pretreated Pd/Al₂O₃ catalyst (containing 1.5 wt. % of Pd) was filled in an upper section of the second reactor. The upper section of the second reactor was heated to the temperature of 130°C, and the lower section thereof was heated to the temperature of 350°C. Thereafter, 1,1,1,2,3-pentafluoropropene and H₂ obtained from the first reactor were introduced to the second reactor for reaction with a molar ratio of H₂ to 1,1,1,2,3-pentafluoropropene of 1:0.97 and a space velocity of 1000 h⁻¹, and products obtained from an outlet of the second reactor were washed by an alkaline and then samples were collected for analysis, results of which are listed in Table 4-2.

**Table 4-2 Data analysis of organic substances at an outlet of a second reactor**

| Components | 2,3,3,3-tetrafluorop ropene | 1,1,1,2,3-pentafluor opropane |
|---|---|---|
| Contents (wt. %) | 61.5 | 38.5 |

### Example 5

The activation of the Cr₂O₃ catalyst and the pretreatment of the Pd/C catalyst were the same as Example 1. 200 mL of the activated Cr₂O₃ catalyst was filled in a lower section of a first reactor (adiabatic reactor made of carbon steel), and 150 mL of the pretreated Pd/C catalyst (containing 1.0 wt. % of Pd) was filled in an upper section of the first reactor. The upper section of the first reactor was heated to the temperature of 150°C, and the lower section of the first reactor was heated to the temperature of 330°C. Hexafluoropropylene and H₂ were introduced to the first reactor at a molar ratio of hexafluoropropylene to H₂ of 1:0.98 at a space velocity of 1000 h⁻¹ for reaction, and products obtained from an outlet of the first reactor were washed by an alkaline and then samples were collected for analysis, results of which are listed in Table 5-1.

**Table 5-1 Data analysis of organic substances at an outlet of a first reactor**

| Components | 1,1,1,2,3-pent afluoropropen e | 1,1,1,2,3,3-he xafluoropropa ne | HFP |
|---|---|---|---|
| Contents (wt. %) | 68.4 | 31.5 | 0.1 |

The activation of the catalyst comprising Cr₂O₃ and ZnO₂ and the pretreatment of the Pd/Al₂O₃ catalyst were the same as Example 1. 200 mL of the activated catalyst comprising Cr₂O₃ and ZnO₂ (90 wt. % of Cr₂O₃ and 10 wt. % of ZnO₂) was filled in a lower section of the second reactor (adiabatic reactor made of stainless steel), and 180 mL of the pretreated Pd/Al₂O₃ catalyst (containing 0.5 wt. % of Pd) was filled in an upper section of the second reactor. The upper section of the second reactor was heated to the temperature of 100°C, and the lower section thereof was heated to the temperature of 450°C. Thereafter, 1,1,1,2,3-pentafluoropropene and H₂ obtained from the first reactor were introduced to the second reactor for reaction with a molar ratio of H₂ to 1,1,1,2,3-pentafluoropropene of 1:0.98 and a space velocity of 1500 h⁻¹, and products obtained from an outlet of the second reactor were washed by an alkaline and then samples were collected for analysis, results of which are listed in Table 5-2.

**Table 5-2 Data analysis of organic substances at an outlet of a second reactor**

| Components | 2,3,3,3-tetrafluorop ropene | 1,1,1,2,3-pentafluor opropane |
|---|---|---|
| Contents (wt. %) | 79.7 | 20.3 |

### Example 6

The activation of the Cr₂O₃ catalyst and the pretreatment of the Pd/C catalyst were the same as Example 1. 200 mL of the activated Cr₂O₃ catalyst was filled in a lower section of a first reactor (adiabatic reactor made of carbon steel), and 150 mL of the pretreated Pd/C catalyst (containing 1.0 wt. % of Pd) was filled in an upper section of the first reactor. The upper section of the first reactor was heated to the temperature of 130°C, and the lower section of the first reactor was heated to the temperature of 400°C. Hexafluoropropylene and H₂ were introduced to the first reactor at a molar ratio of hexafluoropropylene to H₂ of 1:0.98 at a space velocity of 500 h⁻¹ for reaction, and products obtained from an outlet of the first reactor were washed by an alkaline and then samples were collected for analysis, results of which are listed in Table 6-1.

**Table 6-1 Data analysis of organic substances at an outlet of a first reactor**

| Components | 1,1,1,2,3-pent afluoropropen e | 1,1,1,2,3,3-he xafluoropropa ne | HFP |
|---|---|---|---|
| Contents (wt. %) | 74.4 | 25.4 | 0.2 |

The activation of the catalyst comprising Cr₂O₃ and ZnO₂ and the pretreatment of the Pd/Al₂O₃ catalyst were the same as Example 1. 200 mL of the activated catalyst comprising Cr₂O₃ and ZnO₂ (80 wt. % of Cr₂O₃ and 20 wt. % of ZnO₂) was filled in a lower section of the second reactor (adiabatic reactor made of stainless steel), and 180 mL of the pretreated Pd/Al₂O₃ catalyst (containing 0.3 wt. % of Pd) was filled in an upper section of the second reactor. The upper section of the second reactor was heated to the temperature of 100°C, and the lower section thereof was heated to the temperature of 500°C. Thereafter, 1,1,1,2,3-pentafluoropropene and H₂ obtained from the first reactor were introduced to the second reactor for reaction with a molar ratio of H₂ to 1,1,1,2,3-pentafluoropropene of 1:0.95 and a space velocity of 2000 h⁻¹, and products obtained from an outlet of the second reactor were washed by an alkaline and then samples were collected for analysis, results of which are listed in Table 6-2.

**Table 6-2 Data analysis of organic substances at an outlet of a second reactor**

| Components | 2,3,3,3-tetrafluorop ropene | 1,1,1,2,3-pentafluor opropane |
|---|---|---|
| Contents (wt. %) | 84.6 | 15.4 |

### Example 7

The activation of the Cr₂O₃ catalyst and the pretreatment of the Pd/C catalyst were the same as Example 1. 200 mL of the activated Cr₂O₃ catalyst was filled in a lower section of a first reactor (adiabatic reactor made of carbon steel), and 150 mL of the pretreated Pd/C catalyst (containing 0.3 wt. % of Pd) was filled in an upper section of the first reactor. The upper section of the first reactor was heated to the temperature of 100°C, and the lower section of the first reactor was heated to the temperature of 300°C. Hexafluoropropylene and H₂ were introduced to the first reactor at a molar ratio of hexafluoropropylene to H₂ of 1:0.99 at a space velocity of 300 h⁻¹ for reaction, and products obtained from an outlet of the first reactor were washed by an alkaline and then samples were collected for analysis, results of which are listed in Table 7-1.

**Table 7-1 Data analysis of organic substances at an outlet of a first reactor**

| Components | 1,1,1,2,3-pent afluoropropen e | 1,1,1,2,3,3-he xafluoropropa ne | HFP |
|---|---|---|---|
| Contents (wt. %) | 61.4 | 38.5 | 0.1 |

The activation of the catalyst comprising Cr₂O₃ and ZnO₂ and the pretreatment of the Pd/Al₂O₃ catalyst were the same as Example 1. 200 mL of the activated catalyst comprising Cr₂O₃ and ZnO₂ (90 wt. % of Cr₂O₃ and 10 wt. % of ZnO₂) was filled in a lower section of the second reactor (adiabatic reactor made of stainless steel), and 180 mL of the pretreated Pd/Al₂O₃ catalyst (containing 0.5 wt. % of Pd) was filled in an upper section of the second reactor. The upper section of the second reactor was heated to the temperature of 150°C, and the lower section thereof was heated to the temperature of 300°C. Thereafter, 1,1,1,2,3-pentafluoropropene and H₂ obtained from the first reactor were introduced to the second reactor for reaction with a molar ratio of H₂ to 1,1,1,2,3-pentafluoropropene of 1:0.99 and a space velocity of 600 h⁻¹, and products obtained from an outlet of the second reactor were washed by an alkaline and then samples were collected for analysis, results of which are listed in Table 7-2.

**Table 7-2 Data analysis of organic substances at an outlet of a second reactor**

| Components | 2,3,3,3-tetrafluorop ropene | 1,1,1,2,3-pentafluor opropane |
|---|---|---|
| Contents (wt. %) | 62.4 | 37.6 |

### Example 8

The activation of the Cr₂O₃ catalyst and the pretreatment of the Pd/C catalyst were the same as Example 1. 200 mL of the activated Cr₂O₃ catalyst was filled in a lower section of a first reactor (adiabatic reactor made of carbon steel), and 150 mL of the pretreated Pd/C catalyst (containing 0.3 wt. % of Pd) was filled in an upper section of the first reactor. The upper section of the first reactor was heated to the temperature of 120°C, and the lower section of the first reactor was heated to the temperature of 250°C. Hexafluoropropylene and H₂ were introduced to the first reactor at a molar ratio of hexafluoropropylene to H₂ of 1:0.99 at a space velocity of 500 h⁻¹ for reaction, and products obtained from an outlet of the first reactor were washed by an alkaline and then samples were collected for analysis, results of which are listed in Table 8-1.

**Table 8-1 Data analysis of organic substances at an outlet of a first reactor**

| Components | 1,1,1,2,3-pent afluoropropen e | 1,1,1,2,3,3-he xafluoropropa ne | HFP |
|---|---|---|---|
| Contents (wt. %) | 52.7 | 47.2 | 0.1 |

The activation of the catalyst comprising Cr₂O₃ and ZnO₂ and the pretreatment of the Pd/Al₂O₃ catalyst were the same as Example 1. 200 mL of the activated catalyst comprising Cr₂O₃ and ZnO₂ (90 wt. % of Cr₂O₃ and 10 wt. % of ZnO₂) was filled in a lower section of the second reactor (adiabatic reactor made of stainless steel), and 180 mL of the pretreated Pd/Al₂O₃ catalyst (containing 0.3 wt. % of Pd) was filled in an upper section of the second reactor. The upper section of the second reactor was heated to the temperature of 150°C, and the lower section thereof was heated to the temperature of 300°C. Thereafter, 1,1,1,2,3-pentafluoropropene and H₂ obtained from the first reactor were introduced to the second reactor for reaction with a molar ratio of H₂ to 1,1,1,2,3-pentafluoropropene of 1:0.95 and a space velocity of 600 h⁻¹, and products obtained from an outlet of the second reactor were washed by an alkaline and then samples were collected for analysis, results of which are listed in Table 8-2.

**Table 8-2 Data analysis of organic substances at an outlet of a second reactor**

| Components | 2,3,3,3-tetrafluorop ropene | 1,1,1,2,3-pentafluor opropane |
|---|---|---|
| Contents (wt. %) | 56.7 | 43.3 |

### Example 9

The activation of the Cr₂O₃ catalyst and the pretreatment of the Pd/C catalyst were the same as Example 1. 200 mL of the activated Cr₂O₃ catalyst was filled in a lower section of a first reactor (adiabatic reactor made of carbon steel), and 150 mL of the pretreated Pd/C catalyst (containing 0.3 wt. % of Pd) was filled in an upper section of the first reactor. The upper section of the first reactor was heated to the temperature of 80°C, and the lower section of the first reactor was heated to the temperature of 320°C. Hexafluoropropylene and H₂ were introduced to the first reactor at a molar ratio of hexafluoropropylene to H₂ of 1:0.99 at a space velocity of 500 h⁻¹ for reaction, and products obtained from an outlet of the first reactor were washed by an alkaline and then samples were collected for analysis, results of which are listed in Table 9-1.

**Table 9-1 Data analysis of organic substances at an outlet of a first reactor**

| Components | 1,1,1,2,3-pent afluoropropen e | 1,1,1,2,3,3-he xafluoropropa ne | HFP |
|---|---|---|---|
| Contents (wt. %) | 64.1 | 35.8 | 0.1 |

The activation of the catalyst comprising Cr₂O₃ and ZnO₂ and the pretreatment of the Pd/Al₂O₃ catalyst were the same as Example 1. 200 mL of the activated catalyst comprising Cr₂O₃ and ZnO₂ (90 wt. % of Cr₂O₃ and 10 wt. % of ZnO₂) was filled in a lower section of the second reactor (adiabatic reactor made of stainless steel), and 180 mL of the pretreated Pd/Al₂O₃ catalyst (containing 0.5 wt. % of Pd) was filled in an upper section of the second reactor. The upper section of the second reactor was heated to the temperature of 100°C, and the lower section thereof was heated to the temperature of 350°C. Thereafter, 1,1,1,2,3-pentafluoropropene and H₂ obtained from the first reactor were introduced to the second reactor for reaction with a molar ratio of H₂ to 1,1,1,2,3-pentafluoropropene of 1:0.95 and a space velocity of 400 h⁻¹, and products obtained from an outlet of the second reactor were washed by an alkaline and then samples were collected for analysis, results of which are listed in Table 9-2.

**Table 9-2 Data analysis of organic substances at an outlet of a second reactor**

| Components | 2,3,3,3-tetrafluorop ropene | 1,1,1,2,3-pentafluor opropane |
|---|---|---|
| Contents (wt. %) | 58.4 | 41.6 |

## Claims

1. A method for preparing 2,3,3,3-tetrafluoropropene, **characterized in that** the method comprises:
a) introducing hexafluoropropylene and hydrogen to a first reactor to allow hexafluoropropylene to react with hydrogen in the presence of a first catalyst and a second catalyst to obtain a first mixture comprising 1,2,3,3,3-pentafluoropropene, 1,1,1,2,3,3-hexafluoropropane, hydrogen fluoride, and hexafluoropropylene, in which a molar ratio of hexafluoropropylene to hydrogen is between 1: 0.95 and 1: 0.99, a space velocity is between 200 and 1000 h⁻¹, and a reaction temperature is between 50 and 400°C;
b) washing and drying the first mixture obtained from a), and introducing the treated first mixture to a first distillation column to obtain 1,1,1,2,3,3-hexafluoropropane in a bottom of the first distillation column and 1,2,3,3,3-pentafluoropropene and hexafluoropropylene at a top of the first distillation column; recycling the 1,1,1,2,3,3-hexafluoropropane to the first reactor, and introducing the 1,2,3,3,3-pentafluoropropene and the hexafluoropropylene to a second distillation column to yield hexafluoropropylene at a top of the second distillation column and 1,2,3,3,3-pentafluoropropene at a bottom the second distillation column; and recycling the hexafluoropropylene to the first reactor;
c) introducing the 1,2,3,3,3-pentafluoropropene obtained from b) and hydrogen to a second reactor and allowing the 1,2,3,3,3-pentafluoropropene to react with the hydrogen in the presence of a third catalyst and a fourth catalyst, to obtain a second mixture comprising 1,1,1,2,3-pentafluoropropane, 2,3,3,3-tetrafluoropropene, HF, and H₂, in which a molar ratio of the hydrogen to the 1,2,3,3,3-pentafluoropropene is between 1: 0.95 and 1: 0.99, a space velocity is between 300 and 2000 h⁻¹, and a reaction temperature is between 80 and 500°C; and
d) washing and drying the second mixture obtained from c), and introducing the second mixture to a third distillation column to yield 1,1,1,2,3-pentafluoropropane at a bottom of the third distillation column; and recycling the 1,1,1,2,3-pentafluoropropane to the second reactor, to yield 2,3,3,3-tetrafluoropropene at a top of the third distillation column;
wherein:
the first catalyst and the second catalyst in the first reactor are respectively filled in an upper section and a lower section of the first reactor;
the first catalyst in the upper section of the first reactor is Pd/C, and Pd accounts for between 0.1 and 1 wt. %;
the second catalyst in the lower section of the first reactor is chromium oxide;
the third catalyst and the fourth catalyst in the second reactor are respectively filled in an upper section and a lower section of the second reactor;
the third catalyst in the upper section of the second reactor is Pd/Al₂O₃, and Pd accounts for between 0.2 and 1.5 wt. %; and
the fourth catalyst in the lower section of the second reactor comprises between 80 and 90 wt. % of chromium oxide and between 10 and 20 wt. % of zinc oxide.

2. The method of claim 1, **characterized in that** the space velocity in a) is between 400 and 800 h⁻¹, and the reaction temperature is between 100 and 300°C.

3. The method of claim 1, **characterized in that** the space velocity in c) is between 600 and 1500 h⁻¹, and the reaction temperature is between 120 and 400°C.

4. The method of claim 1, **characterized in that** both the first reactor and the second reactor are adiabatic reactors.

## Patentansprüche

1. Verfahren zur Herstellung von 2,3,3,3-Tetrafluorpropen, **dadurch gekennzeichnet, dass** das Verfahren umfasst:
a) Einleiten von Hexafluorpropylen und Wasserstoff in einen ersten Reaktor, um es Hexafluorpropylen zu ermöglichen, sich mit Wasserstoff in Gegenwart eines ersten Katalysators und eines zweiten Katalysators umzusetzen, um eine erste Mischung zu erhalten, die 1,2,3,3,3-Pentafluorpropen, 1,1,1,2,3,3-Hexafluorpropan, Fluorwasserstoff und Hexafluorpropylen umfasst, wobei ein Molverhältnis von Hexafluorpropylen zu Wasserstoff zwischen 1: 0,95 und 1: 0,99 beträgt, eine Raumgeschwindigkeit zwischen 200 und 1.000 h⁻¹ beträgt, und eine Reaktionstemperatur zwischen 50 und 400 °C beträgt;
b) Waschen und Trocknen der ersten Mischung, die aus a) erhalten wurde, und Einleiten der behandelten ersten Mischung in eine erste Destillationskolonne, um 1,1,1,2,3,3-Hexafluorpropan an einem unteren Ende der ersten Destillationskolonne und 1,2,3,3,3-Pentafluorpropen und Hexafluorpropen an einem oberen Ende der ersten Destillationskolonne zu erhalten; Rückführen des 1,1,1,2,3,3-Hexafluorpropans in den ersten Reaktor und Einleiten des 1,2,3,3,3-Pentafluorpropens und des Hexafluorpropens in eine zweite Destillationskolonne, um Hexafluorpropen an einem oberen Ende der zweiten Destillationskolonne und 1,2,3,3,3-Pentafluorpropen an einem unteren Ende der zweiten Destillationskolonne zu erhalten; und Rückführen des Hexafluorpropens in den ersten Reaktor;
c) Einleiten des aus b) erhaltenen 1,2,3,3,3-Pentafluorpropens und von Wasserstoff in einen zweiten Reaktor und es dem 1,2,3,3,3-Pentafluorpropen ermöglichen, dass es sich mit dem Wasserstoff in Gegenwart eines dritten Katalysators und eines vierten Katalysators umsetzt, um eine zweite Mischung zu erhalten, die 1,1,1,2,3-Pentafluorpropan, 2,3,3,3-Tetrafluorpropen, HF und H₂ umfasst, wobei ein Molverhältnis des Wasserstoffs zu dem 1,2,3,3,3-Pentafluorpropen zwischen 1: 0,95 und 1: 0,99 beträgt, eine Raumgeschwindigkeit zwischen 300 und 2.000 h-1 und eine Reaktionstemperatur zwischen 80 und 500 °C beträgt; und
d) Waschen und Trocknen der zweiten Mischung, die aus c) erhalten wurde, und Einleiten der zweiten Mischung in eine dritte Destillationskolonne, um 1,1,1,2,3-Pentafluorpropan an einem unteren Ende der dritten Destillationskolonne zu erhalten; und Rückführen des 1,1,1,2,3-Pentafluorpropans in den zweiten Reaktor, um 2,3,3,3-Tetrafluorpropen an einem oberen Ende der dritten Destillationskolonne zu erhalten;
wobei:
der erste Katalysator und der zweite Katalysator in dem ersten Reaktor jeweils in einen oberen Abschnitt und einen unteren Abschnitt des ersten Reaktors gefüllt sind;
der erste Katalysator in dem oberen Abschnitt des ersten Reaktors Pd/C ist, und Pd zwischen 0,1 und 1 Gew.-% beträgt;
der zweite Katalysator in dem unteren Abschnitt des ersten Reaktors Chromoxid ist;
der dritte Katalysator und der vierte Katalysator in dem zweiten Reaktor jeweils in einen oberen Abschnitt und einen unteren Abschnitt des zweiten Reaktors gefüllt sind;
der dritte Katalysator in dem oberen Abschnitt des zweiten Reaktors Pd/Al₂O₃ ist und Pd zwischen 0,2 und 1,5 Gew.-% beträgt; und
der vierte Katalysator in dem unteren Abschnitt des zweiten Reaktors zwischen 80 und 90 Gew.-% Chromoxid und zwischen 10 und 20 Gew.-% Zinkoxid umfasst.

2. Verfahren nach Anspruch 1, **dadurch gekennzeichnet, dass** die Raumgeschwindigkeit in a) zwischen 400 und 800 h⁻¹ beträgt und die Reaktionstemperatur zwischen 100 und 300 °C beträgt.

3. Verfahren nach Anspruch 1, **dadurch gekennzeichnet, dass** die Raumgeschwindigkeit in c) zwischen 600 und 1.500 h⁻¹ beträgt und die Reaktionstemperatur zwischen 120 und 400 °C beträgt.

4. Verfahren nach Anspruch 1, **dadurch gekennzeichnet, dass** sowohl der erste Reaktor als auch der zweite Reaktor adiabatische Reaktoren sind.

## Revendications

1. Procédé de préparation de 2,3,3,3-tétrafluoropropène, **caractérisé en ce que** le procédé comprend :
a) l'introduction d'hexafluoropropylène et d'hydrogène dans un premier réacteur pour permettre la réaction de l'hexafluoropropylène avec l'hydrogène en présence d'un premier catalyseur et d'un deuxième catalyseur pour obtenir un premier mélange comprenant du 1,2,3,3,3-pentafluoropropène, du 1,1,1,2,3,3-hexafluoropropane, du fluorure d'hydrogène, et de l'hexafluoropropylène, dans lequel rapport molaire de l'hexafluoropropylène sur l'hydrogène est compris entre 1/0,95 et 1/0,99, une vitesse spatiale est comprise entre 200 et 1 000 h⁻¹, et une température de réaction est comprise entre 50 et 400 °C ;
b) le lavage et le séchage du premier mélange obtenu de a), et l'introduction du premier mélange traité dans une première colonne de distillation pour obtenir du 1,1,1,2,3,3-hexafluoropropane dans un fond de la première colonne de distillation et du 1,2,3,3,3-pentafluoropropène et de l'hexafluoropropylène au niveau d'un sommet de la première colonne de distillation ; le recyclage du 1,1,1,2,3,3-hexafluoropropane vers le premier réacteur, et l'introduction du 1,2,3,3,3-pentafluoropropène et de l'hexafluoropropylène dans une deuxième colonne de distillation pour donner l'hexafluoropropylène au niveau d'un sommet de la seconde colonne de distillation et le 1,2,3,3,3-pentafluoropropène au niveau d'un fond de la deuxième colonne de distillation ; et le recyclage de l'hexafluoropropylène vers le premier réacteur ;
c) l'introduction du 1,2,3,3,3-pentafluoropropène obtenu de b) et de l'hydrogène dans un deuxième réacteur et le fait de permettre la réaction du 1,2,3,3,3-pentafluoropropène avec l'hydrogène en présence d'un troisième catalyseur et d'un quatrième catalyseur, pour obtenir un deuxième mélange comprenant du 1,1,1,2,3-pentafluoropropane, du 2,3,3,3-tétrafluoropropène, du HF, et de l'H₂, dans lequel un rapport molaire de l'hydrogène sur le 1,2,3,3,3-pentafluoropropène est compris entre 1/0,95 et 1/0,99, une vitesse spatiale est comprise entre 300 et 2 000 h⁻¹, et une température de réaction est comprise entre 80 et 500 °C ; et
d) le lavage et le séchage du deuxième mélange obtenu de c), et l'introduction du deuxième mélange dans une troisième colonne de distillation pour donner le 1,1,1,2,3-pentafluoropropane au niveau d'un fond de la troisième colonne de distillation ; et le recyclage du 1,1,1,2,3-pentafluoropropane vers le deuxième réacteur, pour donner le 2,3,3,3-tétrafluoropropène au niveau d'un sommet de la troisième colonne de distillation ;
dans lequel :
le premier catalyseur et le deuxième catalyseur dans le premier réacteur sont respectivement remplis dans une section supérieure et une section inférieure du premier réacteur ;
le premier catalyseur dans la section supérieure du premier réacteur est le Pd/C, et le Pd représente de 0,1 à 1 % en poids ;
le deuxième catalyseur dans la section inférieure du premier réacteur est l'oxyde de chrome ;
le troisième catalyseur et le quatrième catalyseur dans le deuxième réacteur sont respectivement remplis dans une section supérieure et une section inférieure du deuxième réacteur ;
le troisième catalyseur dans la section supérieure du deuxième réacteur est le Pd/Al₂O₃, et le Pd représente de 0,2 à 1,5 % en poids ; et
le quatrième catalyseur dans la section inférieure du deuxième réacteur comprend de 80 à 90 % en poids d'oxyde de chrome et de 10 à 20 % en poids d'oxyde de zinc.

2. Procédé selon la revendication 1, **caractérisé en ce que** la vitesse spatiale en a) est comprise entre 400 et 800 h⁻¹, et la température de réaction est comprise entre 100 et 300 °C.

3. Procédé selon la revendication 1, **caractérisé en ce que** la vitesse spatiale en c) est comprise entre 600 et 1 500 h⁻¹, et la température de réaction est comprise entre 120 et 400 °C.

4. Procédé selon la revendication 1, **caractérisé en ce qu'**à la fois le premier réacteur et le deuxième réacteur sont des réacteurs adiabatiques.
